(19) Europäisches Patentamt
European Patent Office
Office européen des brevets

(11) Veröffentlichungsnummer: **0 074 121**
**B1**

(12) **EUROPÄISCHE PATENTSCHRIFT**

(45) Veröffentlichungstag der Patentschrift: **10.04.85**

(51) Int. Cl.⁴: **C 07 D 313/08**

(21) Anmeldenummer: **82108303.7**

(22) Anmeldetag: **29.07.80**

(60) Veröffentlichungsnummer der früheren Anmeldung nach Art. 76 EPÜ:

(54) **2,3,4,5-Tetrahydro-1-benzoxepin-3,5-dion-Derivate und Verfahren zu deren Herstellung.**

(30) Priorität: **02.08.79 DE 2931398**

(43) Veröffentlichungstag der Anmeldung: **16.03.83 Patentblatt 83/11**

(45) Bekanntmachung des Hinweises auf die Patenterteilung: **10.04.85 Patentblatt 85/15**

(84) Benannte Vertragsstaaten: **AT BE CH DE FR GB IT LI LU NL SE**

(56) Entgegenhaltungen:
**CHEMICAL ABSTRACTS, Band 66, Nr. 5, 30. Januar 1967, Seite 1801, Spalte 1, Nr. 18659u, Columbus, Ohio, USA, J.H.P. TYMAN et al.: "Novel preparation of 1-benzoxepins"**
**THE JOURNAL OF ORGANIC CHEMISTRY, Band 42, Nr. 25, 9. Dezember 1977, Seiten 4265-4266, B.K. WASSON et al.: "A synthesis of 6-hydroxy-1-benzoxepin-3,5(2H,AH)-dione"**

(73) Patentinhaber: **Kali-Chemie Pharma GmbH, Hans-Böckler-Allee 20 Postfach 220, D-3000 Hannover 1 (DE)**

(72) Erfinder: **Ohlendorf, Heinrich W., Dipl.-Chem., Dr.rer.nat., Schumachersweg 18, D-3008 Garbsen 2 (DE)**
Erfinder: **Wolf, Klaus Ullrich, Dr.Med.vet., Imkersweg 6, D-3165 Hänigsen (DE)**
Erfinder: **Kaupmann, Wilhelm, Dipl.-Chem., Dr.Ing., St. Ingbert-Weg 9, D-3000 Hannover-Kirchrode (DE)**
Erfinder: **Heinemann, Henning, Dipl.-Chem., Dr.rer.nat., Berglusstrasse 18, D-3000 Hannover 51 (DE)**

(74) Vertreter: **Lauer, Dieter, Dr., c/o Kali-Chemie Aktiengesellschaft Postfach 220, D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft die Herstellung von 2,3,4,5-Tetrahydro-1-benzoxepin-3,5-dionderivaten und nach diesem Verfahren herstellbar neue 2,3,4,5-Tetrahydro-1-benzoxepin-3,5-dionderivate.

Der vorliegenden Erfindung liegt die Aufgabe zugrunde, Zwischenprodukte zur Herstellung neuer Verbindungen mit wertvollen pharmakologischen und therapeutischen Eigenschaften und Verfahren zur Herstellung dieser Zwischenprodukte zu entwickeln.

Das unsubstituierte 2,3,4,5-Tetrahydro-1-benzoxepin-3,5-dion ist aus einer Studie über Umlagerungsreaktionen an Chromonen bekannt, bei der es als ein Hydrolyseprodukt von 2,3-Dihydro-5-hydroxy-3-oxo-1-benzoxepin-4-carboxaldehyd erhalten wurde (s. „Synthesis" [1977], S. 61-63). Ferner wurde das 7-Brom-8-methyl-2,3,4,5-tetrahydro-1-benzoxepin-3,5-dion bei der Cyclisierung von 2-Acetyl-4-brom-5-methylphenoxyessigsäureäthylester zu Benzofuranverbindungen in geringen Mengen als Nebenprodukt aufgefunden (s. Tyman *et al.*, „Tetrahedron Letters", *41* [1966], 4993-4996).

Überraschenderweise wurde gefunden, dass 2,3,4,5-Tetrahydro-1-benzoxepin-3,5-dionderivate nach dem erfindungsgemässen neuen Verfahren in guter Ausbeute hergestellt werden können und wertvolle Zwischenprodukte darstellen zur Herstellung entsprechender 3-Amino-1-benzoxepin-5-(2H)-onderivate, welche eine gute Wirkung auf die Motilität des Magens haben. Diese neuen 2-Amino-1-benzoxepin-5-(2H)-onderivate, ihre pharmakologischen Eigenschaften und ihre Herstellung aus den erfindungsgemässen 3-Oxo-1-benzoxepin-5-(2H)-onen sind in der europäischen Patentanmeldung Nr. 80104466 (Veröffentlichungsnummer 25109) ausführlich beschrieben und beansprucht.

Gegenstand der Erfindung sind daher neue 2,3,4,5-Tetrahydro-1-benzoxepin-3,5-dionderivate der Formel Ia

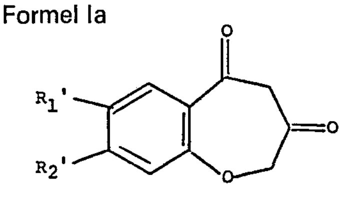

(Ia)

worin $R'_1$ und $R'_2$ unabhängig voneinander jeweils Halogen oder eine Alkyl-, Alkoxy- oder Alkylthiogruppe oder einer der beiden Reste $R'_1$ und $R'_2$ Halogen oder eine Alkyl-, Alkoxy-, Alkylthio-, Trifluormethyl- oder Nitrogruppe und der andere Wasserstoff sein können, wobei der Alkylrest jeweils 1 bis 4 Kohlenstoffatome haben kann, mit der Ausnahme, dass, wenn $R'_1$ Brom ist, $R'_2$ nicht Methyl ist. Die Verbindungen stellen wertvolle neue Zwischenprodukte zur Herstellung von Arzneimitteln dar.

Für die Substituenten $R_1$ und $R_2$ am Phenylring kommen als Halogenatome Fluor-, Chlor-, Brom- oder Jodatome, insbesondere Fluor-, Chlor- oder Bromatome in Frage. Die $C_1$- bis $C_4$-Alkylreste in den Alkyl-, Alkoxy- oder Alkylthiogruppen können gerade oder verzweigt sein, und z.B. Methyl, Äthyl, Propyl, Isopropyl, Butyl, Isobutyl oder tert.-Butyl darstellen. Hierbei sind, insbesondere bei Mehrfachsubstitution am Phenylrest, ein C-Atom enthaltende Gruppen wie Methyl-, Methoxy- oder Methylthiogruppen bevorzugt.

Gegenstand der Erfindung ist ferner ein Verfahren zur Herstellung von 2,3,4,5-Tetrahydro-1-benzoxepin-3,5-dionderivaten der Formel I

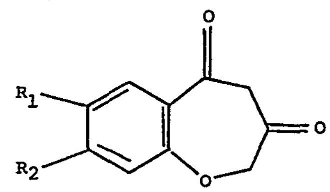

(I)

worin $R_1$ und $R_2$ unabhängig voneinander jeweils Wasserstoff, Halogen oder eine Alkyl-, Alkoxy- oder Alkylthiogruppe bedeuten, wobei der Alkylrest jeweils 1 bis 4 Kohlenstoffatome haben kann, oder einer der beiden Reste $R_1$ oder $R_2$ eine Trifluormethyl- oder Nitrogruppe und der andere Wasserstoff sind, dadurch gekennzeichnet, dass man Verbindungen der Formel II

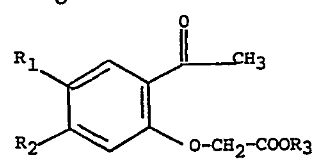

(II)

in welcher $R_1$ und $R_2$ die obige Bedeutung haben und $R_3$ eine gerade oder verzweigte niedermolekulare Alkylgruppe, vorzugsweise eine Methylgruppe, ist, mit einer starken Base aus der Reihe Lithiumhydrid, Natriumhydrid, Lithium-tert.-butylat oder Kalium-tert.-butylat in Gegenwart eines inerten Lösungsmittels bei Temperaturen zwischen −70°C und der Siedetemperatur des Lösungsmittels umsetzt. Als Lösungsmittel eignen sich beispielsweise Dimethylformamid oder Tetrahydrofuran.

Zur Aufarbeitung kann das Reaktionsgemisch mit Eiswasser versetzt und die ausfallende Verbindung der Formel I abgetrennt werden. Man kann aber auch durch Ausfällen der Alkalisalze, insbesondere des Lithiumsalzes, mit einem unpolaren Lösungsmittel, beispielsweise Toluol oder Petroläther, die Verbindungen der Formel I von den Nebenprodukten abtrennen. Aus dem Salz kann die freie Verbindung mittels einer anorganischen oder organischen Säure, beispielsweise einer wässerigen Lösung von Salzsäure, Schwefelsäure oder Essigsäure, freigesetzt werden.

Es ist überraschend, dass unter Verwendung der oben genannten Basen, vorzugsweise Natriumhydrid und Lithium-tert.-butylat, ein Ringschluss der Verbindungen der Formel II zu Verbindungen der Formel I in guten Ausbeuten stattfindet. Es ist nämlich bekannt, dass bei Cyclisierungsversuchen mit 2'-Acetylphenoxyacetaten mittels Natriumäthylat Benzofuranderivate als Hauptreaktionsprodukt erhalten werden und Benzoxepindione gar nicht oder nur in geringen Mengen als Nebenprodukte auftreten (s. „J. Org. Chem.", vol. 42

[1977], S. 4265, sowie „Tetrahedron Letters" [1966], Nr. 41, S. 4995, Abs. 1).

Die für die Herstellung von 2,3,4,5-Tetrahydro-1-benzoxepin-3,5-dionderivaten der Formel I erforderlichen 2'-Acetylphenoxyacetate können bekanntlich aus den 2-Hydroxyacetophenonen in guter Ausbeute erhalten werden, so dass ein einfacher Weg zur Herstellung der in der europäischen Patentanmeldung Nr. 80104466.0 beschriebenen pharmakologisch interessanten 3-Amino-1-benzoxepin-5-(2H)-onderivate gegeben ist.

Die 2,3,4,5-Tetrahydro-1-benzoxepin-3,5-dionverbindungen der Formel I können direkt mit Aminen, z.B. niederen Mono- oder Dialkylaminen oder niederen N-(Dialkyl)alkylendiaminen, umgesetzt werden zu entsprechenden 3-Amino-1-benzoxepin-5-(2H)-onverbindungen. Die Umsetzung kann in an sich bekannter Weise in einem inerten Lösungsmittel erfolgen und durch Zugabe katalytischer Mengen einer Säure begünstigt werden. Die Verbindungen der Formel I können auch zunächst durch Behandlung mit einem Säurechlorid wie Oxalylchlorid in die entsprechenden 3-Halogen-1-benzoxepin-5-(2H)-onverbindungen überführt und diese anschliessend in an sich bekannter Weise mit einem Amin weiter umgesetzt werden.

Die nachstehenden Beispiele dienen zur Erläuterung der Erfindung.

*Beispiel 1:*

Zu einer auf −20°C gekühlten Lösung von 242 g (1 mol) (2'-Acetyl-4'-chlor)phenoxyessigsäuremethylester in 300 ml Dimethylformamid werden 30,1 g (1 mol) Natriumhydrid (80% in Öl) unter Kühlung so in kleinen Portionen zugefügt, dass die Temperatur nicht über −10°C ansteigt. Anschliessend wird noch 45 min bei −15°C gerührt, dann die Lösung vorsichtig in Eiswasser gegossen und einmal mit Toluol extrahiert. Nach Ansäuern der wässerigen Phase wird das ausgefallene Produkt abgesaugt und aus Cyclohexan/Toluol umkristallisiert. Es werden 126 g 2,3,4,5-Tetrahydro-7-chlor-1-benzoxepin-3,5-dion mit Fp. 131-134°C erhalten (60% d.Th.).

*Beispiel 2:*

Zu einer Lösung von 28,7 g (0,1 mol) 2'-Acetyl-4'-bromphenoxyessigsäuremethylester in 150 ml trockenem Tetrahydrofuran werden 8,8 g (0,11 mol) Lithium-tert.-butylat in 50 ml trockenem Tetrahydrofuran unter Kühlung so zugetropft, dass die Temperatur zwischen 25-35°C gehalten wird. Anschliessend wird die Suspension in 400 ml Petroläther gegossen und das ausgefallene Lithiumsalz des 2,3,4,5-Tetrahydro-7-brom-1-benzoxepin-3,5-dions abgesaugt. Dieses Salz wird in ein Gemisch aus 150 ml Wasser und 11 ml Salzsäure (32%) eingetragen. Das ausgefallene Produkt wird abgesaugt, in Dichlormethan gelöst, die Lösung mit gesättigter Kochsalzlösung gewaschen, über Natriumsulfat getrocknet, eingedampft und der Rückstand aus Cyclohexan umkristallisiert. Es wurden 11,7 g (46% d.Th.) 2,3,4,5-Tetrahydro-7-brom-1-benzoxepin-3,5-dion mit Fp. 110-112°C erhalten.

*Beispiel 3:*

Verwendung von 2,3,4,5-Tetrahydro-1-benzoxepin-3,5-dion zur Herstellung einer 3-Amino-1-benzoxepin-5-(2H)-onverbindung.

Eine Lösung von 88 g (0,5 mol) 2,3,4,5-Tetrahydro-1-benzoxepin-3,5-dion und einer Spatelspitze p-Toluolsulfonsäure in 750 ml Toluol wird unter Rühren mit 44 g (0,5 mol) N,N-Dimethyläthylendiamin versetzt und anschliessend bis zur Beendigung der Reaktion bei Raumtemperatur gerührt. Der nach Einengen der Lösung gewonnene Rückstand wird abgesaugt und aus Benzol/Ligroin umkristallisiert. Es werden 108 g (88% d.Th) 3-(β-Dimethylaminoäthylamino)-1-benzoxepin-5-[2H]-on mit Fp. 100-101°C erhalten.

Auf die in den Beispielen 1 und 2 beschriebene Weise erhält man unter Verwendung von Natriumhydrid bzw. Lithium-tert.-butylat in ähnlichen Ausbeuten aus

(2'-Acetyl-4'-methyl)phenoxyessigsäuremethylester,
(2'-Acetyl-5'-methyl)phenoxyessigsäuremethylester,
(2'-Acetyl-5'-chlor)phenoxyessigsäuremethylester,
(2'-Acetyl-4'-fluor)phenoxyessigsäuremethylester,
(2'-Acetyl-4'-methoxy)phenoxyessigsäuremethylester,
(2'-Acetyl-5'-methoxy)phenoxyessigsäuremethylester,
(2'-Acetyl-4',5'-dichlor)phenoxyessigsäuremethylester,
(2'-Acetyl-4'-chlor-5'-methyl)phenoxyessigsäuremethylester,
(2'-Acetyl-4',5'-dimethyl)phenoxyessigsäuremethylester,
(2'-Acetyl-5'-tert.-butyl)phenoxyessigsäuremethylester,
(2'-Acetyl-4'-äthyl)phenoxyessigsäuremethylester,
2'-Acetylphenoxyessigsäuremethylester und anderen

| die Verbindungen: | *Fp. (°C)* |
|---|---|
| 2,3,4,5-Tetrahydro-7-methyl-1-benzoxepin-3,5-dion | 124-127 |
| 2,3,4,5-Tetrahydro-8-methyl-1-benzoxepin-3,5-dion | 97-98 |
| 2,3,4,5-Tetrahydro-8-chlor-1-benzoxepin-3,5-dion | 152-154 |
| 2,3,4,5-Tetrahydro-7-fluor-1-benzoxepin-3,5-dion | 138-140 |
| 2,3,4,5-Tetrahydro-7-methoxy-1-benzoxepin-3,5-dion | 101-102 |
| 2,3,4,5-Tetrahydro-8-methoxy-1-benzoxepin-3,5-dion | 125-127 |
| 2,3,4,5-Tetrahydro-7,8-dichlor-1-benzoxepin-3,5-dion | 168-170 |
| 2,3,4,5-Tetrahydro-7-chlor-8-methyl-1-benzoxepin-3,5-dion | 172-174 |

| die Verbindungen: | *Fp. (°C)* |
|---|---|
| 2,3,4,5-Tetrahydro-7,8-dimethyl-1-benzoxepin-3,5-dion | 117-118 |
| 2,3,4,5-Tetrahydro-8-tert.-butyl-1-benzoxepin-3,5-dion | Öl |
| IR ($CH_2Cl_2$): 1686, 1738 $cm^{-1}$ | |
| 2,3,4,5-Tetrahydro-7-äthyl-1-benzoxepin-3,5-dion | 74-75 |
| 2,3,4,5-Tetrahydro-1-benzoxepin-3,5-dion | 83-86 |
| 2,3,4,5-Tetrahydro-7-nitro-1-benzoxepin-3,5-dion | 138-139 |

## Patentansprüche

1. Verfahren zur Herstellung der 2,3,4,5-Tetrahydro-1-benzoxepin-3,5-dionderivate der Formel (I)

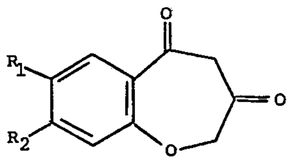

(I)

worin $R_1$ und $R_2$ unabhängig voneinander jeweils Wasserstoff, Halogen oder eine Alkyl-, Alkoxy- oder Alkylthiogruppe bedeuten, wobei der Alkylreste jeweils 1 bis 4 Kohlenstoffatome haben kann, oder einer der beiden Reste $R_1$ oder $R_2$ die Trifluormethyl- oder Nitrogruppe und der andere Wasserstoff sind, dadurch gekennzeichnet, dass man Verbindungen der Formel (II)

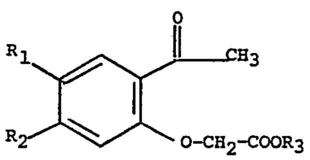

(II)

in welcher $R_1$ und $R_2$ die obige Bedeutung haben und $R_3$ eine gerade oder verzweigte niedermolekulare Alkylgruppe, vorzugsweise eine Methylgruppe, ist, mit einer starken Base aus der Reihe Lithiumhydrid, Natriumhydrid, Lithium-tert.-butylat in Gegenwart eines inerten Lösungsmittels bei Temperaturen zwischen −70°C und der Siedetemperatur des Lösungsmittels umsetzt.

2. 2,3,4,5-Tetrahydro-1-benzoxepin-3,5-dionderivate der Formel (Ia)

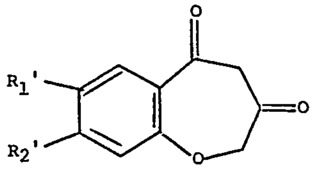

(Ia)

worin $R'_1$ und $R'_2$ unabhängig voneinander jeweils Halogen oder eine Alkyl-, Alkoxy- oder Alkylthiogruppe oder einer der beiden Reste $R'_1$ oder $R'_2$ Halogen oder eine Alkyl-, Alkoxy-, Alkylthio-, Trifluormethyl- oder Nitrogruppe und der andere Wasserstoff sein können, wobei der Alkylrest jeweils 1 bis 4 Kohlenstoffatome haben kann, mit der Ausnahme, dass, wenn $R'_1$ Brom ist, $R'_2$ nicht Methyl ist.

3. Verwendung der 2,3,4,5-Tetrahydro-1-benzoxepin-3,5-dionderivate der Formel (I)

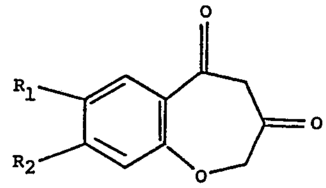

(I)

worin $R_1$ und $R_2$ unabhängig voneinander jeweils Wasserstoff, Halogen oder eine Alkyl-, Alkoxy- oder Alkylthiogruppe bedeuten, wobei der Alkylrest jeweils 1 bis 4 Kohlenstoffatome haben kann, oder einer der beiden Reste $R_1$ und $R_2$ die Trifluormethyl- oder Nitrogruppe und der andere Wasserstoff sind, als Zwischenprodukte zur Herstellung von 3-Amino-1-benzoxepin-5-(2H)-onverbindungen.

## Claims

1. Method for the production of the 2,3,4,5-tetrahydro-1-benzoxepine-3,5-dione-derivatives of the Formula (I)

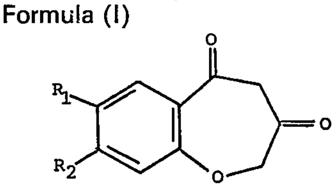

(I)

in which $R_1$ and $R_2$ independently of each other in each case signify hydrogen, halogen or an alkyl-, alkoxy- or alkylthio-group, where the alkyl radical in each case may have 1 to 4 carbon atoms, or one of the two radicals $R_1$ or $R_2$ is the trifluoromethyl- or nitro-group and the other is hydrogen, characterized in that compounds of the Formula (II)

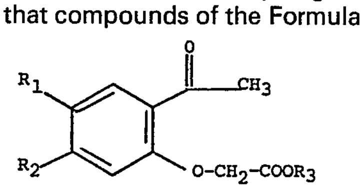

(II)

in which $R_1$ and $R_2$ have the above meaning and $R_3$ is a straight or branched lower alkyl group, preferably a methyl group, are reacted with a strong base from the series lithium hydride, sodium hydride, lithium-tert.-butylate in the presence of an inert solvent at temperatures between −70°C and the boiling temperature of the solvent.

2. 2,3,4,5-Tetrahydro-1-benzoxepine-3,5-dione-derivatives of the Formula (Ia)

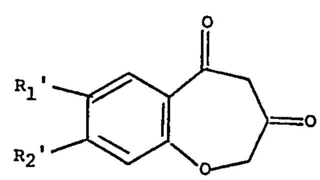

(Ia)

in which $R'_1$ and $R'_2$ independently of each other in each case may be halogen or an alkyl-, alkoxy- or alkylthio-group, or one of the two radicals $R'_1$

and $R'_2$ may be halogen or an alkyl-, alkoxy-, alkylthio-, trifluoromethyl- or nitro-group and the other may be hydrogen, where the alkyl radical in each case may have 1 to 4 carbon atoms, with the exception that when $R'_1$ ist bromine, $R'_2$ is not methyl.

3. The use of the 2,3,4,5-tetrahydro-1-benzoxepine-3,5-dione-derivatives of Formula (I)

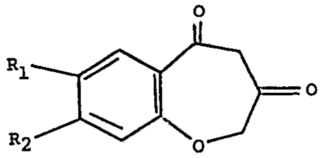

(I)

in which $R_1$ and $R_2$ independently of each other in each case signify hydrogen, halogen or an alkyl-, alkoxy- or alkylthio-group, where the alkyl radical in each case may have 1 to 4 carbon atoms, or one of the two radicals $R_1$ or $R_2$ is the trifluoromethyl- or nitro-group and the other is hydrogen, as intermediates for the production of 3-amino-1-benzoxepine-5-(2H)-one compounds.

**Revendications**

1. Procédé de préparation des dérivés de la tétrahydro-2,3,4,5 benzoxépine-1 dione-3,5 de formule (I):

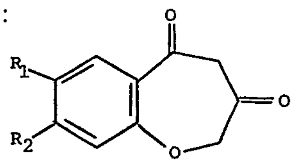

(I)

dans laquelle $R_1$ et $R_2$ peuvent représenter, indépendamment l'un de l'autre, chacun un atome d'hydrogène ou d'halogène ou un groupe alkyle, alcoxy ou alkylthio, le radical alkyle pouvant avoir à chaque fois 1 à 4 atomes de carbone, ou bien l'un des deux radicaux $R_1$ ou $R_2$ représentant le groupe trifluorométhyle ou nitro et l'autre représentant un atome d'hydrogène, procédé caractérisé en ce que l'on fait réagir des composés de formule (II):

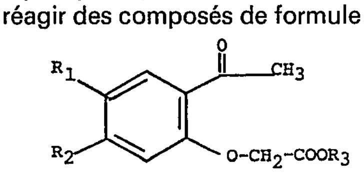

(II)

dans laquelle $R_1$ et $R_2$ ont le sens ci-dessus, et $R_3$ est un groupe alkyle à bas poids moléculaire, linéaire ou ramifié, de préférence un groupe méthyle avec une base forte choisie dans la série hydrure de lithium, hydrure de sodium, tertiobutylate de lithium, en présence d'un solvant inerte, à des températures comprises entre −70°C et la température d'ébullition du solvant.

2. Dérivés de la tétrahydro-2,3,4,5 benzoxépine-1 dione-3,5 de formule (Ia):

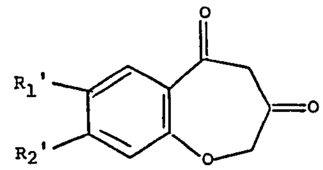

(Ia)

dans laquelle $R'_1$ et $R'_2$ peuvent représenter, indépendamment l'un de l'autre, chacun un atome d'halogène ou un groupe alkyle, alcoxy ou alkylthio ou bien l'un des deux radicaux $R'_1$ ou $R'_2$ peut être un atome d'halogène ou un groupe alkyle, alcoxy, alkylthio, trifluorométhyle ou nitro et l'autre peut être un atome d'hydrogène, le radical alkyle pouvant comporter à chaque fois 1 à 4 atomes de carbone, étant bien entendu que lorsque $R'_1$ représente un atome de brome, $R'_2$ n'est pas un groupe méthyle.

3. Utilisation des dérivés de tétrahydro-2,3,4,5 benzoxépine-1 dione-3,5 de formule (I):

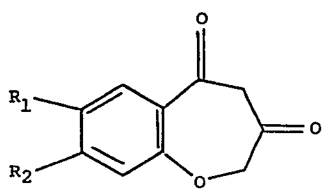

(I)

dans laquelle $R_1$ et $R_2$ peuvent représenter, indépendamment l'un de l'autre, chacun un atome d'hydrogène ou d'halogène ou un groupe alkyle, alcoxy ou alkylthio, le radical alkyle pouvant comporter à chaque fois 1 à 4 atomes de carbone, ou bien l'un des deux radicaux $R_1$ ou $R_2$ représente un groupe trifluorométhyle ou nitro et l'autre un atome d'hydrogène comme produits intermédiaires pour la préparation de composés dérivant de l'amino-3 benzoxépine-1 one-5 (2H).